# EUROPEAN PATENT SPECIFICATION

(11) **EP 0 579 708 B1**
(45) Date of publication and mention of the grant of the patent: **04.11.1998**
(21) Application number: 92909072.8
(22) Date of filing: 10.04.1992
(51) Int. Cl.: C12P 7/64, D21C 3/00

(54) **LIPASE-CATALYZED ESTER HYDROLYSIS**
LIPASEKATALYSIERTE ESTERHYDROLYSE
HYDROLYSE D'ESTERS CATALYSES EN PRESENCE D'UNE LIPASE

(30) Priority: 10.04.1991 EP 91610031; 16.04.1991 EP 91610034
(43) Date of publication of application: 26.01.1994
(73) Proprietor: NOVO NORDISK A/S, 2880 Bagsvaerd (DK); NIPPON PAPER INDUSTRIES CO., LTD., Kita-ku, Tokyo 115-91 (JP)
(72) Inventor: FUJITA, Yuko, Komae-shi, Tokyo 201 (JP); AWAJI, Haruo, Ohmiya-shi, Saitama-ken 330 (JP); SHIMOTO, Hidesato, Funabashi-shi, Chiba-ken 274 (JP); SHARYOU, Masaki, Matsudo-shi, Chiba-ken 270 (JP); HELDT-HANSEN, Hans, Peter, DK-2830 Virum (DK)
(74) Representative: Knudsen, Sten Lottrup
(86) International application number: DK9200115
(87) International publication number: WO9218638

(56) References cited:
- EP-A- 0 374 700
- WO-A-88/02775
- WO-A-89/01032
- WO-A-90/10687
- CHEMICAL ABSTRACTS, Volume 100, No. 2, 9 January 1984, (Columbus, Ohio, US), JAMES C. LINDEN et al., "Pretreatment of Crop Cellulosics", page 115, Abstract 8780h; & PROC.- INT. SYMP. ETHANOL. BIOMASS., 1983, 161-205.
- CHEMICAL ABSTRACTS, Volume 114, No. 5, 4 February 1991, (Columbus, Ohio, US), RUGHANI JAGDISH et al., "The Use of Lewis Acids During Rapid Steam Hydrolysis", page 123, Abstract 45246y; & J. WOOD CHEM. TECHNOL., 1990, 10(4), 515-530.

## Description

### TECHNICAL FIELD

This invention relates to a process for hydrolysis of water-insoluble ester in the presence of a lipase derived from a strain of *Humicola, Candida antarctica* or *Pseudomonas cepacia* at a pH in the range 3-7, and particularly to such a process for hydrolysis of resin in pulp. It also relates to a method of increasing the rate of hydrolysis of water-insoluble ester in the presence of such a lipase.

### BACKGROUND ART

It is known that lipases can be used with advantage for efficient hydrolysis of water-insoluble esters, particularly triglycerides at acid pH. It is also known that some types of pulp made from wood have a high resin content. The resin can create disturbances in the process of pulp manufacture and may also have a negative effect on the properties of the final pulp product. Resin of wood material contains considerable amounts of triglycerides and can be hydrolyzed by lipase.

It is the object of this invention to provide an improved process for ester hydrolysis, applicable to hydrolysis of resin ester, using the above lipases.

### STATEMENT OF THE INVENTION

We have found that, surprisingly, addition of aluminium salt significantly increases the hydrolysis rate of esters in the presence of the above lipases at acid pH. A strong increase of fat hydrolysis (from c. 50% to c. 87% hydrolysis) was found with as little as 10 mM Al⁺⁺⁺.

T. Nishio *et al., Agric. Biol. Chem.,* **51** (1), 181-186, 1987, in Table II shows that the activity of lipase from *Pseudomonas fragi* at pH 9.0 is lowered to 94% by incubation with 1x10⁻³ M of Al³⁺. In contrast to this, we have surprisingly found a significant increase of the activity of the above lipases by addition of lower concentrations of Al⁺⁺⁺ at acidic pH.

EP 374,700 discloses the treatment of resin with a lipase from *Candida cylindracea, Aspergillus niger, Pseudomonas fluorescens* or *Geotrichum candidum* in the presence of aluminium sulfate, and TAPPI Papermakers' Conference, 24 April 1990, Atlanta, describes the hydrolysis of resin by a lipase from *Candida cylindracea* in the presence of aluminium sulfate. However, the effect of the aluminium salt on the hydrolysis rate was not described.

Accordingly, the invention provides a process for hydrolysis of water-insoluble ester in the presence of a lipase derived from a strain of *Humicola, Candida antarctica* or *Pseudomonas cepacia* at a pH in the range 3-7, characterized by being carried out in the presence of a soluble aluminium salt at a concentration of 1-50 mM as Al⁺⁺⁺.

The invention also provides a method of increasing the rate of hydrolysis at pH 3-7 of water-insoluble ester in the presence of a lipase derived from a strain of *Humicola, Candida antarctica* or *Pseudomonas cepacia* by incorporation of a soluble aluminium salt at a concentration of 1-50 mM as Al⁺⁺⁺.

### DETAILED DESCRIPTION OF THE INVENTION

### Process conditions

Typical process conditions are pH 3-6, particularly 4-5.5, a temperature from ambient to 70°C, particularly 30-60°C, and reaction times of 0.5-3 hours.

Examples of suitable enzymes are lipases derived from strains of *Pseudomonas cepacia, Candida antarctica* (e.g. lipase A or B, see WO 88/02775) and *Humicola* (especially *H. brevispora, H. lanuginosa, H. brevis var. thermoidea* and *H. insolens).*

The amount of lipase will typically correspond to a lipase activity of 1,000-100,000 LU/kg dry matter or 50-5,000 LU/litre (LU = Lipase Unit, defined in WO 89/04361).

The aluminium salt used in the invention may be any salt that is soluble at the conditions of the process. Conveniently, aluminium sulfate or aluminium chloride can be used.

A suitable concentration for achieving increased hydrolysis rate is 1-50 mM as Al⁺⁺⁺, preferably 2-20 mM.

### Ester hydrolysis

The process of the invention can be used for any lipase-catalyzed hydrolysis of water-insoluble esters, particularly triglycerides.

Thus, the process of the invention may be used for fat hydrolysis in the production of fatty acids, glycerides and/or glycerol from fat or oil. This may be a liquid at ambient temperature, such as soy bean oil and many other oils and many other oils, or it may be a high melting fat, such as beef tallow.

### Hydrolysis of resin esters

The process of the invention is particularly applicable to the hydrolysis of resin esters during a pulping or paper-making process, e.g. to avoid pitch troubles such as paper contamination or paper breaks.

The process of the invention may be applied to any pitch-containing pulp, especially to pulps with a considerable content of triglycerides and other esters from pitch. Examples are pulps produced by mechanical pulping, alone or combined with a gentle chemical treatment, such as GW (Ground Wood), TMP (Thermo Mechanical Pulp) and CTMP (Chemical Thermo Mechanical Pulp).

Hydrolysis of esters in pitch according to the invention can be done in the pulping or stock preparation section, where addition of aluminium sulfate (alum) is particularly advantageous since it can also act as a retention or flocculation aid. The pulp typically has a consistency of 0.2-5% dry substance.

To avoid break-down of the fibre structure in the pulp, cellulase side-activities should be essentially absent, preferably below 1000 EGU/kg of pulp dry matter. Cellulase activity in EGU units is determined as follows:

A substrate solution is prepared, containing 34.0 g/l CMC (Hercules 7 LFD) in 0.1 M phosphate buffer at pH 6.0. The enzyme sample to be analyzed is dissolved in the same buffer. 5 ml substrate solution and 0.15 ml enzyme solution are mixed and transferred to a vibration viscosimeter (e.g. MIVI 3000 from Sofraser, France), thermostated at 40°C. One Endo-Glucanase Unit (EGU) is defined as the amount of enzyme that reduces the viscosity to one half under these conditions. The amount of enzyme sample should be adjusted to provide 0.01-0.02 EGU/ml in the reaction mixture.

### EXAMPLES

### EXAMPLE 1

### Effect of various Al⁺⁺⁺ concentrations

Ground wood pulp was treated with *Humicola* lipase in the presence of varying amounts of aluminium sulfate. After the reaction, the degree of hydrolysis was determined by quantitative TLC using latroscan™.

Conditions were 4.0% pulp slurry, pH 4.5, 40°C, 2 hours reaction time, enzyme dosage 60 LU/g. The dosage of aluminium salt is given as Al₂(SO₄)₃ in % w/w of dry pulp. Results:

| % Al₂(SO₄)₃ | % hydrolysis |
|---|---|
| 0 | 50.3 |
| 0.5 | 73.2 |
| 2.5 | 87.0 |
| 3 | 85.6 |
| 6 | 100 |
| 30.75 | 90.5 |
| 64.0 | 96.7 |

These results demonstrate increasing hydrolysis rate up to an aluminium dosage of about 6% relative to dry matter, i.e. up to about 2.5 g/l or about 10 mM as Al⁺⁺⁺.

### EXAMPLE 2

A number of different microbial lipases were tested in the same manner as in Example 1 using 0 and 6% Al₂(SO₄)₃ (approx. 2.5 g/l or 10 mM Al⁺⁺⁺). The dosage of each lipase was adjusted so as to obtain a comparable degree of hydrolysis in each experiment without Al⁺⁺⁺. Results (% hydrolysis):

| Origin of lipase | Without Al⁺⁺⁺ | With Al⁺⁺⁺ |
|---|---|---|
| *Humicola lanuginosa* | 56.5 | 78.8 |
| *Candida antarctica* | | |
| lipase A | 60.5 | 96.4 |
| *Pseudomonas cepacia* | 59.7 | 94.2 |

A strong activation of the lipase effect is seen with the lipases tested, both of yeast and bacterial origin.

## Claims

1. A process for the hydrolysis of a water-insoluble ester in the presence of a lipase derived from a strain of *Humicola, Candida antarctica* or *Pseudomonas cepacia* at a pH in the range 3-7, characterized by being carried out in the presence of a soluble aluminium salt at a concentration of 1-50 mM as Al⁺⁺⁺.

2. A process according to claim 1, wherein the concentration of the aluminium salt is 2-20 mM as Al⁺⁺⁺.

3. A process according to claim 1 or 2, wherein the aluminium salt is aluminium chloride or aluminium sulfate.

4. A process according to any of claims 1-3, wherein the pH is in the range 3-6, preferably 4-5.5

5. A process according to any of claims 1-4 wherein the lipase concentration corresponds to an activity of 1-100 Kilo Lipase Units (KLU) per kg of dry matter.

6. A process according to any of claims 1-5 for hydrolysis of esters in pitch during a pulping or paper-making process.

7. A process according to claim 6, whereby the cellulase activity is below 1000 Endo-Glucanase Units (EGU) per kg.

8. A method of increasing the rate of hydrolysis at pH 3-7 of water-insoluble ester in the presence of a lipase derived from a strain of *Humicola, Candida antarctica* or *Pseudomonas cepacia* by incorporation of a soluble aluminium salt at a concentration of 1-50 mM as Al⁺⁺⁺.

## Patentansprüche

1. Verfahren zur Hydrolyse eines wasserunlöslichen Esters in Gegenwart einer Lipase, die von einem Stamm von Humicola, Candida antarctica oder Pseudomonas cepacia abgeleitet ist, bei einem pH-Wert im Bereich von 3-7, dadurch gekennzeichnet, daß das Verfahren in Gegenwart eines löslichen Aluminiumsalzes in einer Konzentration von 1-50 mM, angegeben als Al⁺⁺⁺, durchgeführt wird.

2. Verfahren nach Anspruch 1, wobei die Konzentration des Aluminiumsalzes 2-20 mM, angegeben als Al⁺⁺⁺, beträgt.

3. Verfahren nach Anspruch 1 oder 2, wobei es sich beim Aluminiumsalz um Aluminiumchlorid oder Aluminiumsulfat handelt.

4. Verfahren nach einem der Ansprüche 1-3, wobei der pH-Wert im Bereich von 3-6 und vorzugsweise von 4-5,5 liegt.

5. Verfahren nach einem der Ansprüche 1-4, wobei die Lipasekonzentration einer Aktivität von 1-100 Kilo-Lipase-Einheiten (KLU) pro kg Trockenbestandteile entspricht.

6. Verfahren nach einem der Ansprüche 1-5 zur Hydrolyse von Estern in Harz (pitch) während eines Zellstoffaufschließoder Papierherstellungsverfahrens.

7. Verfahren nach Anspruch 6, wobei die Cellulase-Aktivität unter 1000 Endo-glucanase-Einheiten (EGU) pro kg liegt.

8. Verfahren zur Erhöhung der Geschwindigkeit der Hydrolyse eines wasserunlöslichen Esters beim pH-Wert 3-7 in Gegenwart einer Lipase, die von einem Stamm von Humicola, Candida antarctica oder Pseudomonas cepacia abgeleitet ist, durch Einverleiben eines löslichen Aluminiumsalzes in einer Konzentration von 1-50 mM in Form von Al⁺⁺⁺.

## Revendications

1. Un processus pour l'hydrolyse d'un ester insoluble dans l'eau en présence d'une lipase dérivée d'une souche de *Humicola, Candida antarctica* ou *Pseudomonas cepacia* à un pH entre 3 et 7, caractérisé en ce qu'il est mis en oeuvre en présence d'un sel d'aluminium soluble à une concentration de 1 à 50 mM en Al⁺⁺⁺.

2. Un processus selon la revendication 1, dans lequel la concentration du sel d'aluminium est de 2 à 20 mM en Al⁺⁺⁺.

3. Un processus selon la revendication 1 ou 2, dans lequel le sel d'aluminium est le chlorule d'aluminium ou le sulfate d'aluminium.

4. Un processus selon l'une des revendications 1 à 3, où le pH est entre 3 et 6, de préférence entre 4 et 5,5.

5. Un processus selon l'une des revendication 1 à 4, dans lequel la concentration en lipase correspond à une activité de 1 à 100 Kilo Unités Lipase (KLU) par kg de matière sèche.

6. Un processus selon l'une des revendications 1 à 5, pour l'hydrolyse d'esters en poix pendant un processus de réduction en pâte ou de fabrication de papier.

7. Un processus selon la revendication 6, dans lequel l'activité de cellulase est inférieure à 1000 Unités Endo-Glucanase (EGU) par kg.

8. Un procédé pour augmenter la vitesse d'hydrolyse à un pH de 3 à 7 d'un ester insoluble dans l'eau en présence d'une lipase dérivée d'une souche de *Humicola, Candida antarctica* ou *Pseudomonas cepacia* par incorporation d'un sel d'aluminium soluble à une concentration de 1 à 50 mM en Al⁺⁺⁺.
